# EUROPEAN PATENT SPECIFICATION

(11) **EP 1 811 866 B1**
(45) Date of publication and mention of the grant of the patent: **26.09.2018**
(21) Application number: 05803077.6
(22) Date of filing: 15.11.2005
(51) Int. Cl.: A23L 33/105, A23L 33/15, A61P 21/00

(54) **THE USE OF ANTI-OXIDANT COMPOUNDS FOR MUSCLE DAMAGE**
VERWENDUNG VON ANTIOXIDANTEN ZUR MUSKELVERLETZUNG
UTILISATION DE COMPOSÉS ANTIOXYDANTS POUR LÉSIONS MUSCULAIRES

(30) Priority: 16.11.2004 EP 04105813
(43) Date of publication of application: 01.08.2007
(73) Proprietor: DSM IP Assets B.V., 6411 TE Heerlen (NL)
(72) Inventor: KIES, Arie Karst, NL-2642 AC Pijnacker (NL); RIETJENS, Saskia Johannes, NL-6221 GJ Maastricht (NL); HAENEN, Guido Rembertus Michiel Marie, NL-6245 EV Eijsden (NL); BAST, Aalt, NL-3862 XG Nijkerk (NL)
(74) Representative: Dux, Roland
(86) International application number: PCT/EP2005/055987
(87) International publication number: WO 2006/053872

(56) References cited:
- WO-A-03/082259
- WO-A-2004/032873
- US-A- 6 149 610
- US-B1- 6 399 116
- DATABASE WPI Section Ch, Week 199406 Derwent Publications Ltd., London, GB; Class B07, AN 1994-047185 XP002321441 & SU 1 787 420 A1 (MOSC MED ACAD) 15 January 1993 (1993-01-15)
- PATENT ABSTRACTS OF JAPAN vol. 007, no. 263 (C-196), 24 November 1983 (1983-11-24) & JP 58 146241 A (NIPPON YUSHI KK), 31 August 1983 (1983-08-31)
- TUTOUR B LE ET AL: "ANTIOXIDATIVE ACTIVITIES OF OLEA EUROPAEA LEAVES AND RELATED PHENOLIC COMPOUNDS" PHYTOCHEMISTRY, PERGAMON PRESS, GB, vol. 31, no. 4, 1992, pages 1173-1178, XP000565805 ISSN: 0031-9422

## Description

### Field of the invention

The present invention relates to the use of anti-oxidant compounds to retain or restore muscle health. These compounds can be used in the manufacture for a composition to retain or restore muscle health, thereby preventing and/or treating muscle damage due to exercise. More specifically, such composition includes food, beverages, supplements and feed.

Athletes take regular intense and/or prolonged muscular exercise. Studies suggest that during strenuous exercise, generation of reactive oxygen species (ROS) is elevated to a level that overwhelms tissue antioxidant defence systems. The result is oxidative stress, which may cause damage to tissues, for example muscle tissue. In general a good muscular condition is of imperative importance for their performance, especially the skeletal muscles. Exercise can thus impair muscle health, resulting in for example fatigue, lower endurance, loss of function. The use of anti-oxidant supplements has been proposed to prevent such damage (Dietary anti-oxidants and exercise, Journal of Sports Sciences, 2004, 22, 81-94, Powers et al.)

US-B1-6 399 116 describes compositions (beverage, food, or nutritional supplement) comprising salidroside and tyrosol with additional antioxidant (vitamin C) for treating muscle fatigue. SU 1 787 420 describes nutritional supplements comprising oleuropein and hydroxytyrosol which can cross the blood:brain barrier and protect the cerebral tissue against oxidative phenomema, particularly neurodegenerative diseases. Not all anti-oxidants, however, are suitable to recover muscular damage. Studies have showed that some anti-oxidants hardly give any effect. This can have several reasons. Firstly, the bioavailability of anti-oxidants can be low. The uptake of the anti-oxidants by the intestines is then relatively low, resulting in the need of a high amount of anti-oxidant to become effective. Secondly, not all anti-oxidants can pass the membrane of the muscle cells. Furthermore, some anti-oxidants are depleted upon use, also necessitating a relatively high amount of anti-oxidant intake to see an effect. This is not desirable, not only from a health perspective (many metabolites in the body) but also from cost perspective.

Some anti-oxidants are known to be regenerated in a so-called anti-oxidant network, for example regeneration of vitamin E by vitamin C. In vivo studies with respect to vitamin E, however, have not shown any significant effects on anti-oxidant markers.

It is the object of the invention to provide a component suitable for muscle recovery, which can take part in the anti-oxidant network having a good bioavailability.

The invention is defined by the claims. Surprisingly, has been found that an edible compound according to formula (1) can be used in the treatment of muscle damage due to exercise. 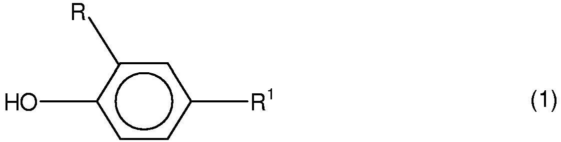 wherein:
R can be H, OH or CH₃O;
R¹ can be H or (CH₂)ₙOR²
wherein: n is 1, 2 or 3
   R² is H, COCH₃ or 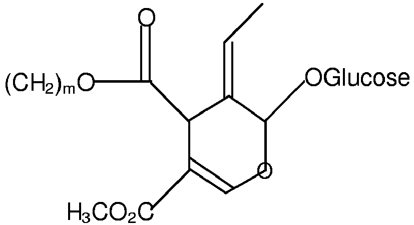 wherein m is 1, 2 or 3
with the proviso that R and R1 are not H at the same time.

Examples of suitable compounds according to formula (1) are hydroxytyrosol and its acetate, oleuropein, tyrosol and its acetate, homovanilic alcohol, and/or cathechol. Also mixtures of these compounds can be used.

It has surprisingly been found that this compound can be regenerated by use of vitamin C. This makes the compound according to formula (1) suitable to be used in the manufacture for a composition to prevent or treat muscle damage due to oxidative stress.

Preferably, R is OH. More preferably at least hydroxytyrosol or its acetate is used in the composition. Hydroxytyrosol has the additional advantage that it is amphiphilic. It can be dissolved in both aqueous and fatty compositions, for example in intracellular and extracellular fluids and membranes.

The compound according to formula 1 can be used for the manufacture of a composition for treating or preventing muscle damage due to exercise. This composition does not comprise salidroside.

Another aspect of the invention is the use according to the invention, wherein the component suitable for muscle recovery is used in a nutritional product suitable for human consumption. Any nutritional product can be suitable, for example food, beverage or dietary supplement. The nutritional product is not a chewing gum.

Preferably, the anti-oxidant compound according to formula (1) is present in sports nutritional products for intake by athletes. It is known that athletes can benefit from particular foods or food ingredients beyond the recommended dietary guidelines for the general population. The term 'sports nutritional products' is herein used to indicate any food or beverage especially adapted for athletes.

There are several types of sports nutrition suitable for the purpose of the invention, for example specific dietary supplements, sport drinks or sports food.

The sports drinks can be hypotonic, hypertonic or isotonic. Sports drinks can be available in liquid form, as concentrates or as powder (to be dissolved in a liquid, as for example water). The sports food can be in the form of bars, tablets and gels. The dietary supplement can be in the form of a pill or a capsule.

The nutrition can further comprise usual additives, for example sweeteners, flavors, sugar, fat, emulgators, preservatives. The nutrition can also comprise other active components, such as (hydrolysed) proteins as described in WO02/45524. Also other anti-oxidants can be present in the nutrition, for example flavonoids, carotenoids, ubiquinones, rutin, lipoic acid, catalase, glutatione (GSH) and vitamins, such as for example C and E or their precursors. The combination of a compound according to formula 1 and vitamin C or its precursor has shown a synergistic effect. Preferably, hydroxytyrosol or its acetate is combined with vitamin C. A composition comprising a compound according to formula 1 and vitamin C is novel and is another aspect of the invention.

The compound according to formula 1 needs to be present in an effective amount. Generally between 1 mg and 3 gram of the compound is needed per serving to assort an effect, preferably 10mg to 1 gram per serving. This depends on a number of factors, such as weight, age, dietary habits and exercise intensity. The effect of an anti-oxidant can be measured via certain markers. In muscle exercise both lipid oxidation and protein oxidation occur. Suitable markers to be measured for the amount of lipid oxidation are for example malondialdehyde (MDA) and isoprotanes. A suitable maker for the amount of protein oxidation are for example protein carbonyls.

The nutrition comprising the compound according to formula (1) can be eaten before, during or after the exercise. In case it is used before exercise, it is preferably eaten about one hour before. In case it is used after exercise, it is preferably eaten within one hour thereafter, more preferably immediately after the exercise.

In addition to nutritional products suitable for consumption of humans, it is also possible to use the compound according to formula (1) in feed for animals including pet food. It is then especially suitable for animals, which are employed for their muscular force, for example (race) horses or dogs (i.e race dogs or sleigh dogs).

The invention is hereafter elucidated with the following non-limiting examples.

### Examples

### Example 1: Organ bath experiments

Male Lewis rats (age between 12 and 14 weeks old) were used. After decapitation, the diaphragm was rapidly excised, and small strips (around 2 mm width and 1 cm long) were cut and subsequently mounted in thermostated organ baths, containing Krebs buffer gassed with a mixture of 95% O₂ and 5% CO₂, pH 7.4. Each diaphragm strip was connected vertically to an isometric transducer. The composition of the Krebs buffer was (mM): NaCl (117.5), KCl (5.6), MgSO₄ (1.18), CaCl₂ (2.5), NaHPO₄ (1.28), NaHCO₃ (25) and glucose (5.5). During the experiment, the buffer was changed every 15 minutes. Field stimulation was created along the entire length of each muscle strip with platinum electrodes.

At the beginning of the experiment, the strips were washed during 45 minutes at room temperature to minimize temperature dependent deterioration. Thereafter, the water bath was turned on at 37°C (15 minutes before the start of the experiments). Each strip was first adjusted to its optimal length (Lo) using twitch contractions (1 Hz). The pulse duration was always 10 ms.

First the Pt (twitch tension at a stimulation of 1 Hz) and Po (maximal specific tension at a stimulation of 100 Hz, 250 ms duration) were determined. Thereafter, the response of the diaphragm muscle strips to increasing stimulus frequencies was assessed at Lo by the application of 10, 20, 33, 50 and 100 Hz-pulses applied in 250 ms trains. A 2-min recovery period was used between contractions. At the completion of the force-frequency protocol, a fatigue protocol was applied. The strips were stimulated for 6 minutes at 5 Hz.

After this stimulation protocol, hydroxytyrosol (HT) or control (vehicle, ethanol) was incubated for 5 minutes. Subsequently, hydrogen peroxide (H₂O₂) or control (MilliQ) was incubated for 10 minutes. After that were again determined: Pt, Po, force-frequency and fatigue.

Hydroxytyrosol was purchased from Cayman Chemical, Ann Arbor, USA. Hydrogen peroxide was obtained from Sigma, St. Louis, USA. All other chemicals were of analytical grade purity.

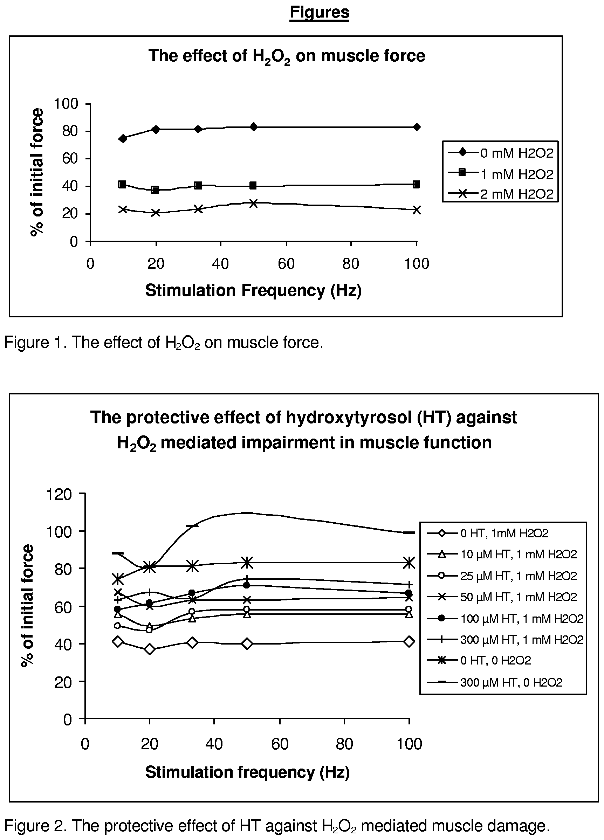

Figure 1 shows the effect of H₂O₂ on muscle force. The H₂O₂ dose-dependently reduces the force at all applied stimulation frequencies, compared with the control. Data are expressed as means, based on at least duplicate measurements.
Figure 2 shows the protective effect of HT against H₂O₂ mediated muscle damage. HT protects against H₂O₂ mediated muscle damage at all stimulation frequencies (10, 20, 33, 50 and 100 Hz). 300 µM HT without H₂O₂ incubation even preserves muscle function. Data are expressed as means, based on at least duplicate measurements.

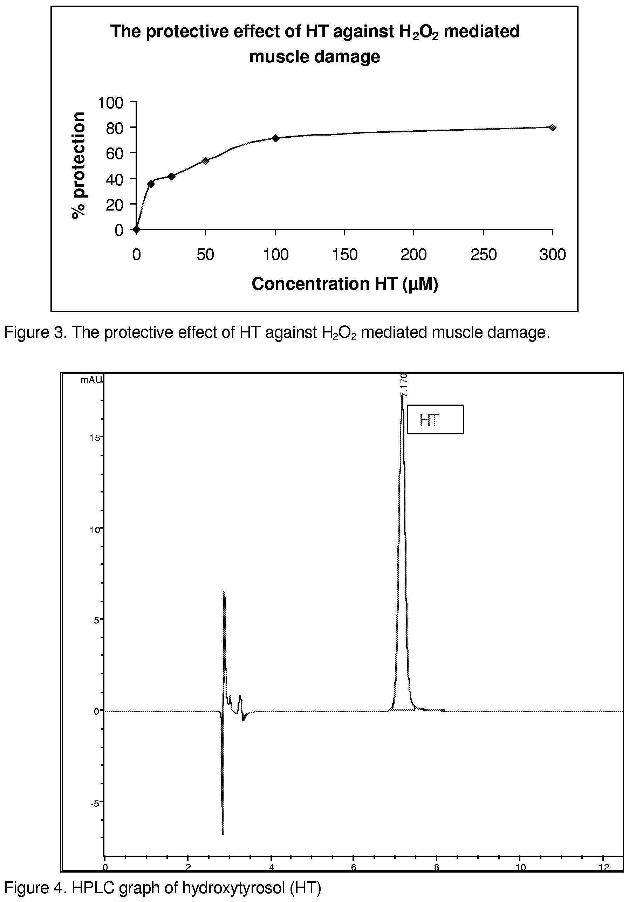

Figure 3 shows the protective effect of HT against H₂O₂ mediated muscle damage. HT dose-dependently protects against the decline in force induced by H₂O₂. The stimulation frequency was 50 Hz and the concentration H₂O₂ was 1 mM. Data are expressed as means, based on at least 2 duplicate measurements.

### Example 2: Scavenging experiments and comparison to lipoic acid.

### Materials

Hydroxytyrosol was obtained from Cayman Chemical, Ann Arbor, USA. Tyrosol and KO₂ were purchased from Fluka, Buchs, Switzerland. Homovanillic alcohol, 2-methoxyphenol, phenol, and dihydrorhodamine-123 (DHR-123) were obtained from Sigma, St. Louis, USA. Catechol was obtained from Janssen Chimica, Geel, Belgium. Lipoic acid was purchased from Asta Medicac AG, Frankfurt, Germany. Nitrogen monoxide was purchased from AGA, Hamburg, Germany. All other chemicals were of analytical grade purity.

### ONOOH scavenging

The protection against ONOOH induced DHR-123 oxidation of the compounds was measured as described by Kooy et al (Peroxynitrite-mediated oxidation of dihydrorhodamine 123. Free Radic Biol Med, 16;149-56). In short, 5 µM DHR-123 and various concentrations of a scavenger were incubated in 100 mM sodium phosphate buffer (pH 7.4) at 37°C. Subsequently, ONOOH was added by pipetting a 10 µl aliquot into the tube while rapid vortexing, reaching a final concentration of 0.7 µM. Fluorescence measurements were performed with excitation and emission wavelengths of respectively 500 and 536 nm. The effects are expressed as the concentration of the scavenger giving 50% inhibition of the oxidation of DHR-123 (IC50).

### O₂•-scavenging

O₂•-were generated by the reaction of xanthine (150 µM) and xanthine oxidase (XO) (5.5 mU/ml) in 50 mM potassium phosphate buffer (pH 7.8). O₂•- radicals were detected by nitroblue tetrazolium (NBT, 50 µM). NBT undergoes reduction by _{O2}•- to its formazan. The rate of this reduction was monitored spectrophotometrically at 560 nm during 2 minutes. Various concentrations of a scavenger were added and incubated at 37°C. The reaction was started by the addition of XO. The O₂•-scavenging potential is expressed as the concentration of the scavenger giving a 50% decrease in the reduction of NBT (IC50). To adjust for a possible inhibitory effect of the scavenger on the activity of XO, the highest concentration of the scavenger was incubated at 37°C and subsequently the rate of the urate formation was measured at 293 nm during 2 minutes.

Results of the scavenging tests are shown below in table 1.

**Table 1. The ONOO- and O₂•- scavenging activities of the tested compounds. Shown are IC50 values ± SEM (in µM), n=3.**

| Compound tested | ONOO⁻ | O₂•⁻ |
|---|---|---|
| Hydroxytyrosol | 3.6 ± 0.2 | 2.3 ± 0.3 |
| Oleuropein | 2.3 ± 0.5 | 6.0 ± 0.5 |
| Tyrosol | 99 ± 16 | >200 |
| Homovanillic alcohol | 6.6 ± 0.2 | >200 |
| Lipoic acid (comparative exp.) | >200 | >100 |

### Example 3: The regeneration of hydroxytyrosol (HT) by ascorbate

The oxidation of HT (Cayman Chemical, Ann Arbor, USA) was performed at 37°C in a 145 mM phosphate buffer, pH 7.4, containing 50 µM HT and 5 U/ml tyrosinase (Sigma, St. Louis, USA). The incubation of tyrosinase and HT leads to the formation of a quinone (HTQ). Reactions were monitored spectrophotometrically and by high performance liquid chromatography (HPLC). Spectra were recorded at a scanspeed of 240 nm/min. HPLC analysis of the incubation mixtures was performed using a Supelcosil LC318 column. The column was eluted with distilled water containing 0.1% (v/v) trifluoroacetic acid and 5% (v/v) acetonitrile with a flow rate of 1 ml per minute. Diode array detection (DAD) at 280 nm was used. The ability of ascorbate to react with HTQ was determined as follows. First, HTQ was formed during two and a half minute. Subsequently, 150 µM ascorbate (Sigma, St. Louis, USA) was added to the HTQ and the mixture was immediately injected by hand into the HPLC at three minutes.

Figure 4 shows the results of the HPLC measurement of HT only (control).

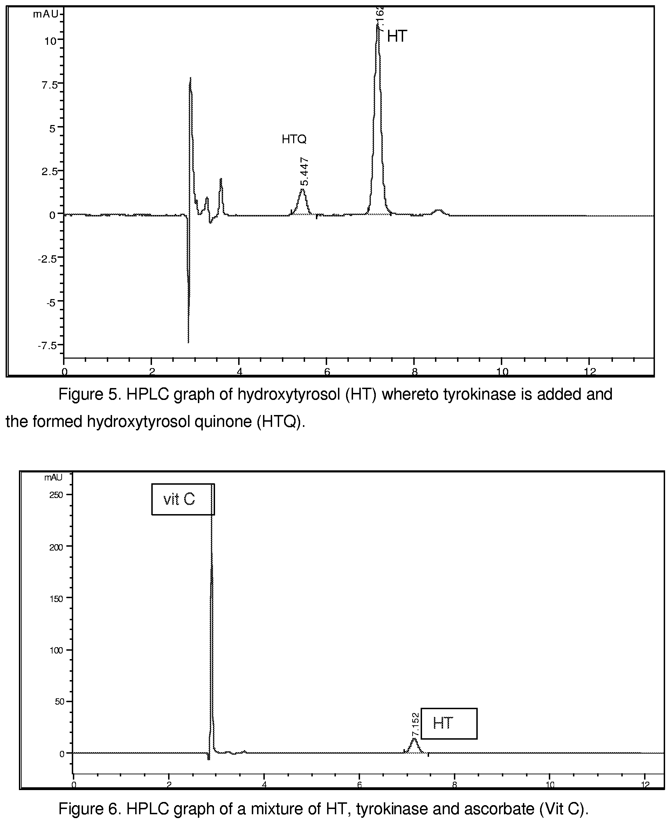

Figure 5 shows the effect of the addition of tyrokinase to HT, thereby showing formation of its quinone (control).
Figure 6 shows the effect of the addition of ascorbate to a mixture of HT and tyrokinase.
This shows that HT is formed again and no HTQ is present any more after three minutes. Regeneration of HT by ascorbate has taken place (example).

### Example 4: Cookie recipe comprising hydroxytyrosol.

Active ingredients: Hydroxytyrosol 10-50 mg/ per serving (typical 30 g)

| Ingredient | (g) |
|---|---|
| Wheat flour | 41.0 |
| Sugar | 20.5 |
| Fat/Butter | 20.5 |
| Whole egg (liquid) | 18.0 |
| Lemon flavour | q.s. |
| Baking agent | q.s. |

All ingredients are added slowly under mixing to form a sweet short pastry. Afterwards, the pastry is kept cool (4°C) for at least 2 hours before flattening the pastry to a thickness of approx. 5 mm. Pieces are cut out and brushed with egg yolk on the surface before baking. Baking can take place for 15 minutes in a fan oven at 180 °C.

## Claims

1. Composition comprising edible compound of formula (1) 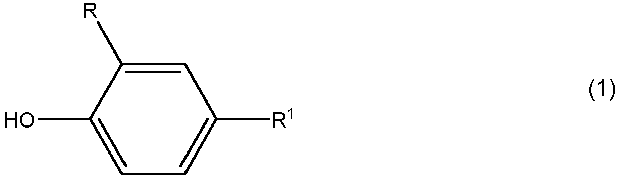 wherein:
R can be H, OH or CH₃O;
R¹ can be H, (CH₂)ₙOR²
wherein: n is 1, 2 or 3
R² is H, COCH₃ or 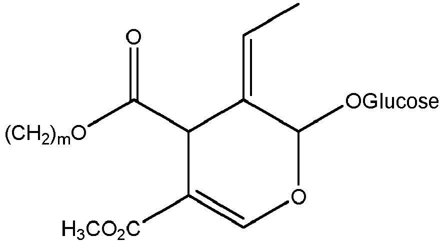
wherein m is 1, 2 or 3
with the proviso that R and R¹ are not H at the same time;
for use in the treatment of muscle damage due to exercise; with the proviso that the composition is not a chewing gum and that the composition does not contain salidroside;
**characterized in that** compound of formula (1) is hydroxytyrosol or its acetate.

2. Composition according to claim 1, **characterized in that** it further comprises vitamin C.

3. Composition according to claim 1 or 2, **characterized in that** the composition is a sports nutritional product for intake by athletes.

4. Composition according to claim 3, **characterized in that** sports nutritional product is a dietary supplement, sport drinks or sports food.

5. Composition according to any of the preceding claims 1-4 **characterized in that** compound of formula (1) is present in an amount of between 1 mg and 3 g, preferably 10 mg to 1 g, per serving.

## Patentansprüche

1. Zusammensetzung, umfassend eine essbare Verbindung der Formel (1) 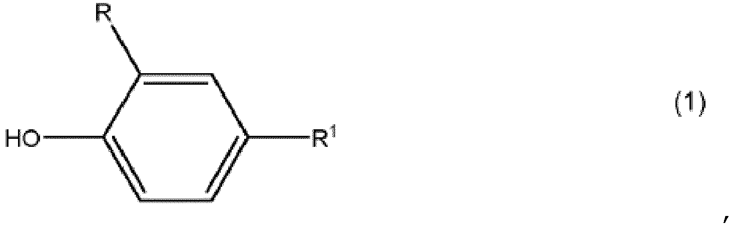 wobei:
R für H, OH oder CH₃O stehen kann,
R¹ für H, (CH₂)ₙOR² stehen kann,
wobei: n für 1, 2 oder 3 steht,
R² für H, COCH₃ oder 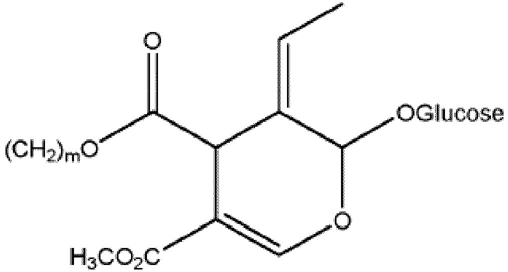 steht, wobei m für 1, 2 oder 3 steht,
mit der Maßgabe, dass R und R¹ nicht gleichzeitig für H stehen,
zur Verwendung bei der Behandlung von Muskelschäden aufgrund körperlicher Betätigung,
mit der Maßgabe, dass es sich bei der Zusammensetzung nicht um ein Kaugummi handelt und dass die Zusammensetzung kein Salidrosid enthält,
**dadurch gekennzeichnet, dass** es sich bei der Verbindung der Formel (1) um Hydroxytyrosol oder dessen Acetat handelt.

2. Zusammensetzung nach Anspruch 1, **dadurch gekennzeichnet, dass** sie weiterhin Vitamin C umfasst.

3. Zusammensetzung nach Anspruch 1 oder 2, **dadurch gekennzeichnet, dass** es sich bei der Zusammensetzung um ein Sporternährungsprodukt zur Einnahme durch Athleten handelt.

4. Zusammensetzung nach Anspruch 3, **dadurch gekennzeichnet, dass** es sich bei dem Sporternährungsprodukt um ein Nahrungsergänzungsmittel, Sportgetränke oder Sportnahrung handelt.

5. Zusammensetzung nach einem der vorhergehenden Ansprüche 1-4, **dadurch gekennzeichnet, dass** die Verbindung der Formel (1) in einer Menge zwischen 1 mg und 3 g, vorzugsweise 10 mg bis 1 g, pro Portion vorliegt.

## Revendications

1. Composition comprenant un composé comestible de formule (1) 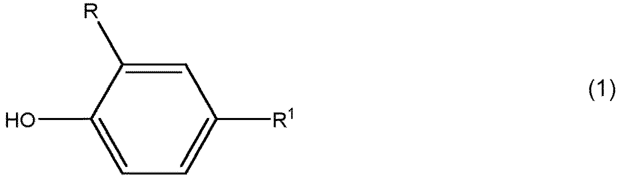 dans laquelle
R peut être H, OH ou CH₃O ;
R¹ peut être H, (CH₂)ₙOR²
où n vaut 1, 2 ou 3
R² est H, COCH₃ ou 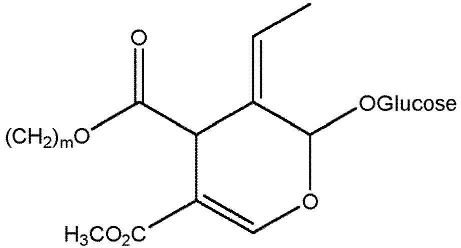 où m vaut 1, 2 ou 3
à condition que R et R¹ ne soient pas H en même temps ; pour une utilisation dans le traitement de lésions musculaires dues à l'exercice ;
à condition que la composition ne soit pas un chewing-gum et que la composition ne contienne pas de salidroside ;
**caractérisée en ce que** le composé de formule (1) est l'hydroxytyrosol ou son acétate.

2. Composition selon la revendication 1, **caractérisée en ce qu'**elle comprend en outre de la vitamine C.

3. Composition selon la revendication 1 ou 2, **caractérisée en ce que** la composition est un produit nutritionnel pour sportifs destiné à être consommé par des athlètes.

4. Composition selon la revendication 3, **caractérisée en ce que** le produit nutritionnel pour sportifs est un complément diététique, une boisson pour sportifs ou un aliment pour sportifs.

5. Composition selon l'une quelconque des revendications 1-4 précédentes, **caractérisée en ce que** le composé de formule (1) est présent selon une quantité comprise entre 1 mg et 3 g, préférablement allant de 10 mg à 1 g, par portion.
